(19) Europäisches Patentamt
European Patent Office
Office européen des brevets

(11) EP 3 427 720 B1

(12) EUROPEAN PATENT SPECIFICATION

(45) Date of publication and mention of the grant of the patent:
**22.05.2024 Bulletin 2024/21**

(21) Application number: **17181366.0**

(22) Date of filing: **14.07.2017**

(51) International Patent Classification (IPC):
***A61K 8/06*** (2006.01) ***A61K 8/41*** (2006.01)
***A61Q 5/08*** (2006.01) ***A61Q 5/10*** (2006.01)
***A61K 8/22*** (2006.01)

(52) Cooperative Patent Classification (CPC):
**A61Q 5/10; A61K 8/06; A61K 8/22; A61K 8/41; A61Q 5/08**

# (54) AQUEOUS BLEACHING AND/OR DYEING COMPOSITION FOR KERATIN FIBERS, PROCESS, KIT AND USE THEREOF

WÄSSRIGE BLEICH- UND/ODER FÄRBEZUSAMMENSETZUNG FÜR KERATINFASERN, VERFAHREN, KIT UND VERWENDUNG DAVON

COMPOSITION AQUEUSE DE BLANCHIMENT ET/OU DE TEINTURE POUR FIBRES KÉRATINIQUES, PROCÉDÉ, KIT ET UTILISATION DE CELUI-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**16.01.2019 Bulletin 2019/03**

(73) Proprietor: **Kao Germany GmbH**
**64297 Darmstadt (DE)**

(72) Inventors:
- **Meulbrouck, Celine**
**64297 Darmstadt (DE)**
- **NÖCKER, Bernd**
**64297 DARMSTADT (DE)**

(74) Representative: **Grit, Mustafa**
**Kao Germany GmbH**
**Pfungstädter Strasse 98-100**
**64297 Darmstadt (DE)**

(56) References cited:
**EP-A1- 1 714 677**
**EP-A1- 2 177 203**
**EP-A1- 2 272 496**
**EP-A1- 3 040 065**
**WO-A1-2017/041908**
**DE-A1- 2 717 538**
**DE-A1-102010 063 369**
**US-A- 5 180 399**
**US-A- 5 520 706**
**US-A- 5 833 966**
**US-A1- 2014 230 163**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

The present invention relates to a bleaching and/or dyeing composition for keratin fibers, preferably human keratin fibers, more preferably human hair. Furthermore, a process, kit-of-parts, and use of the composition are disclosed.

Ammonia-based hair bleaching and/or dyeing compositions and processes are commonly known to consumers with the disadvantage of experiencing a noticeable and disliked ammonia smell. Moreover, despite the strong typical olfactory experience, the bleaching and/or coloring result remains in some cases unsatisfactory because ammonia evaporates over process time and consequently the pH of the hair color shifts to a less effective value. Additionally, ammonia-based bleaching and/or dyeing compositions are known to confer a certain degree of damage to keratin fibers. Hence, mildness to the hair and its perception of the customer is an important factor for such compositions. As alternative to ammonia-based bleaching and/or dyeing systems, organic alkanol amines were introduced into the field of hair treatments. Alkanol amines are either used in combination with ammonia or without ammonia. The advantage of such organic amines is the absence of the unfavourable ammonia smell. However, alkanol amine-based blaching and/or dyeing systems still have performance challenges to overcome.

Apart from consumer experience and performance challenges, there are other technical reasons which render organic amines incompatible with certain hair color compounds, such as direct hair dyes or oxidation sensitive compounds.

In EP2476405 and EP2931227 reduction of ammonia smell was addressed wherein either ammonia was replaced by an organic amine or a specific emulsion was formulated to reduce ammonia evaporation. However, both solutions are not satisfactory because organic amine salts commonly deliver a weaker performance than ammonia and a high amount of fatty compounds may lead to highly viscous compositions which lack good applicability on hair.

EP2177203 discloses an oxidizing composition comprising 0.2% by weight of monoethanolamine at a pH of 3. As this composition does not achieve the effect of the present invention, it is disclaimed from the scope. Thus, the composition of the present invention is different from the one comprising 2.5% by weight hydrogen peroxide, 0.2% by weight Polyquaternium 87, 0.2% by weight monoethanolamine, citric acid q.s. to pH 3.0, 0.3% by weight PEG-40-Hydrogenated castor oil, 0.3% by weight fragrance, and water q.s. to 100.

US2008/0060142 and US2005/0125913 disclose oxidizing compositions comprising triethanolamine at a concentration of 0.7% by weight. However, the documents are silent on the pH of the compositions and, therefore, are distinctly different from the present invention. US5180399A discloses acidic compositions comprising triethanolamine.

In summary of the prior art, none of the documents disclosed a satisfactory solution to reducing ammonia smell while maintaining the performance of ammonia-based products. Moreover, achieving increased hair lightening with organic amines over process time as well as mildness to the hair are not addressed. After extensive research, inventors of the present invention have found that the presence of certain organic amine salts in the oxidizing composition solves the problems of above. Therefore, the first object of the present invention is an aqueous cosmetic composition as defined in claim 1 for bleaching and/or dyeing of keratin fibers, preferably human keratin fibers, more preferably human hair characterized in that it comprises an organic alkyl and/or alkanol amine salt according to the general structure

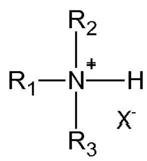

wherein R1, R2, and R3 are independently selected from H, linear C1-C6 alkyl which may be substituted with one hydroxyl group, or branched C3-C12 alkyl or alkanol, wherein at least one of R1, R2, or R3 is different from H, and X- is an anion, wherein the composition has a pH in the range of 1 to 5,

and wherein the composition is different from the one comprising 2.5% by weight hydrogen peroxide, 0.2% by weight Polyquaternium 87, 0.2% by weight monoethanolamine, citric acid q.s. to pH 3.0, 0.3% by weight PEG-40-Hydrogenated castor oil, 0.3% by weight fragrance, and water q.s. to 100.

The second object of the present invention is a process for bleaching and/or dyeing of keratin fibers, preferably human keratin fibers, more preferably human hair, characterized in that it comprises the following steps:

a) mixing the composition according to any of the claims 1 to 10 with a composition comprising an alkalizing agent to form a ready-to-use mixture having a pH in the range of 7 to 12,
b) applying the mixture onto keratin fibers and leaving it for 1 min to 45 min,
c) rinsing-off the mixture,
d) optionally shampooing the keratin fibers,

e) optionally drying the keratin fibers.

The third object of the present invention is a use of the composition as defined above for dyeing and/or bleaching keratin fibers, preferably human keratin fibers, more preferably human hair.

Further object of the present invention is a kit-of-parts comprising in a separately packed container the composition as defined above and in at least one other separately packed container a composition comprising an alkalizing agent.

The organic alkyl and/or alkanol amine salt according to the structure above is selected from mono- and/or diethanolamine, butyl ethanolamine, butyl diethanolamine, dibutyl ethanolamine, methylethanolamine, triethanolamine, N-lauryl diethanolamine, diisopropanolamine, dimethyl isopropanolamine, isopropanolamine, triisopropanolamine, isobutanolamine.

The counterion X- is selected from chloride and/or hydrogen chloride, nitrate, sulphate, phosphate, hydrogenphosphate, dihydrogenphosphate, citrate, acetate, sulphite, benzoate, salicylate.

The most preferred organic amine salt is mono- or diethanolamine hydrochloride. The total concentration of organic amine salts in the composition in the range of 0.5 % to 15% by weight, preferably 0.5% to 12% by weight, more preferably 1% to 10% by weight, calculated to the total of the composition.

In one embodiment of the present invention, the composition may further comprise one or more salt(s) of $NH_4^+$.

The one or more salt(s) of $NH_4^+$ according to the present invention is/are selected from ammonium acetate, ammonium benzoate, ammonium carbamate, ammonium chloride, ammonium ferric pentetate, ammonium fluoride, ammonium iodide, ammonium bromide, ammonium lactate, ammonium nitrate, ammonium phosphate, diammonium phosphate, triammonium phosphate, ammonium propionate, ammonium sulphate, ammonium sulphite, ammonium thiocyanate, ammonium xylenesulfonate, ammonium citrate, diammonium citrate, triammonium citrate. The preferred salt of $NH_4^+$ is selected from ammonium sulphate, ammonium chloride, ammonium acetate, and mono-, di- and/or triammonium citrate.

The most preferred salt of $NH_4^+$ is ammonium sulphate.

Salt(s) of $NH_4^+$ are present in the composition at the total concentration of 0.01 % to 3% by weight, more preferably 0.05% to 1% by weight, further more preferably 0.1% to 0.5% by weight, calculated to the total of the composition.

In the most preferred embodiment of the present invention the composition is free of salts of $NH_4^+$.

The composition according to the present invention comprises hydrogen peroxide at a total concentration of in the range of 1% to 20% by weight, preferably in the range of 1% to 12% by weight, more preferably in the range of 3% to 9% by weight, calculated to the total of the composition.

The composition of the present invention has a pH in the range of 1 to 5, preferably 1.5 to 5, more preferably in the range of 1.8 to 4. In principle, the pH of the composition can be adjusted with any organic and/or inorganic acid(s) or base or their mixtures. Suitable acids are phosphoric acid, hydrochloric acid as the inorganic ones and to the organic acids the well-known citric acid and lactic acid, glycolic acid, glyoxylic acid, hydroxyacrylic acid, glyceric acid, malic acid and tartaric acid and of the dicarboxylic acids are malonic acid, succinic acid, glutaric acid, adipic acid, maleic acid, fumaric acid and phtalic acid. Suitable bases are sodium hydroxide or potassium hydroxide. The most preferred acid is phosphoric acid.

The composition is in the form of an emulsion. For formation of emulsion, the composition comprises lipophilic compounds selected from natural and/or vegetable oils, petrolatum-based compounds, linear or branched, saturated or unsaturated fatty alcohols with C12 to C22, and fatty acid esters consisting of linear or branched, saturated or unsaturated fatty acids with C12 to C22 being esterified with linear or branched primary alcohols with C3 to C12.

Suitable natural and/or vegetable oils are olive oil, almond oil, avocado oil, wheatgerm oil, and castor oil.

Suitable petrolatum-based compounds are liquid paraffins, especially paraffinum perliquidum and paraffinum subliquidum, and mineral oil, in particular white mineral oil.

Suitable comprises fatty compounds selected from linear or branched, saturated or unsaturated fatty alcohols with C12 to C22 are lauryl alcohol, tridecyl alcohol, myristyl alcohol, pentadecyl alcohol, cetyl alcohol, palmitoleyl alcohol, heptadecyl alcohol, stearyl alcohol, nonadecyl alcohol, arachidyl alcohol, behenyl alcohol, and/or their mixtures.

Suitable examples for fatty acid esters consisting of linear or branched, saturated or unsaturated fatty acids with C12 to C22 being esterified with linear or branched primary alcohols with C3 to C18 are octyl palmitate, isocetyl palmitate, isopropyl palmitate, octyl stearate, oleyl oleate, and myristyl myristate, as well as their mixtures.

The composition may also comprise lipophilic ingredients such as silicones for example linear polysiloxanes such as dimethicones with various consistency and dimethiconols, aminated silicones with primary, secondary, tertiary or quaternary ammonium groups such as amodimethicone, polysilicone 9, and quaternium 80, cyclic silicones such as cyclomethicones, arylated silicones such as phenyl trimethicone; C10- to C36-fatty acid triglycerides, as well as their mixtures.

Total concentration of these lipophilic compounds is in the range of 0.1% to 20% by weight, preferably from

1% to 15% by weight, and more preferably from 2% to 10% by weight, calculated to the total of the composition.

The composition further comprises one or more surfactants selected from cationic surfactants and/or non-ionic surfactants and/or anionic surfactants and/or zwitterionic/amphoteric surfactants, preferably non-ionic surfactants.

Suitable non-ionic surfactants are in general all commonly known non-ionic surfactants available on the market.

Suitable nonionic surfactants are alkyl polyglycosides according to the general structure:

$$R^{23}O(R^{24}O)_tZ_x$$

Wherein Z denotes a reducing carbohydrate with $C_5$ to $C_6$, $R^{23}$ is an alkyl group with $C_8$ to $C_{18}$, $R^{24}$ is ethyl or propyl, t ranges from 0 to 10, and x ranges from 1 to 5. Suitable compounds according to this structure are $C_9$-$C_{11}$ alkylpolyglycoside, the structures disclosed in EP-A 70 074, and JP 2015-123019A.

The preferred compound according to the structure of above is decyl glucoside.

Suitable examples for non-ionic surfactants are fatty alcohol ethoxylates of the following general structure

$$R^{18}(OCH_2CH_2)_{n4}\ OH$$

wherein $R^{18}$ is straight or branched, saturated or unsaturated alkyl chain which may be synthetic or natural with a C chain length in the range of 8 to 40, preferably 9 to 30 and more preferably 9 to 24 and n4 is a number in the range of 5 to 40, preferably 9 to 30.

Non-limiting suitable examples of the fatty alcohol ethoxylates are C9-11 Pareth-6, C9-11 Pareth-8, C9-15 Pareth-8, C11-13 Pareth-9, C11-13 Pareth-10, C11-15 Pareth-5, C11-15 Pareth-7, C11-15 Pareth-9, C11-15 Pareth-12, C11-15 Pareth-15, C11-15 Pareth-20, C11-15 Pareth-30, C11-15 Pareth-40, C11-21 Pareth-10, C12-13 Pareth-5, C12-13 Pareth-6, C12-13 Pareth-7, C12-13 Pareth-9, C12-13 Pareth-10, C12-13 Pareth-15, C12-13 Pareth-23, C12-14 Pareth-5, C12-14 Pareth-7, C12-14 Pareth-9, C12-14 Pareth-11, C12-14 Pareth-12, C12-15 Pareth-5, C12-15 Pareth-7, C12-15 Pareth-9, C12-15 Pareth-10, C12-15 Pareth-11, C12-15 Pareth-12, C12-16 Pareth-5, C12-16 Pareth-7, C12-16 Pareth-9, C13-15 Pareth-21, C14-15 Pareth-7, C14-15 Pareth-8, C14-15 Pareth-11, C14-15 Pareth-12, C14-15 Pareth-13, C20-22 Pareth-30, C20-40 Pareth-10, C20-40 Pareth-24, C20-40 Pareth-40, C20-40 Pareth-95, C22-24 Pareth-33, Beheneth-5, Beheneth-10, Beheneth-15, Beheneth-20, Beheneth-25, Beheneth-30, Ceteareth-5, Ceteareth-6, Ceteareth-7, Ceteareth-10, Ceteareth-11, Ceteareth-12, Ceteareth-15, Ceteareth-20, Ceteareth-25, Ceteareth-30, Ceteareth-35, Ceteareth-40, Laureth-5, Laureth-10, Laureth-15, Laureth-20, Laureth-25, Laureth-30, Laureth-40, Myreth-5, Myreth-10, Ceteth-5, Ceteth-10, Ceteth-15, Ceteth-20, Ceteth-25, Ceteth-30, Ceteth-40, Oleth-5, Oleth-10, Oleth-15, Oleth-20, Oleth-25, Oleth-30, Oleth-40, Steareth-5, Steareth-10, Steareth-15, Steareth-20, Steareth-25, Steareth-30, Steareth-35, and Steareth-40. They may also be comprised in the compositions as a mixture of more than one surfactant.

Further suitable nonionic surfactants are polypropylene glycol ethers of fatty alcohol according to general structure

$$R^{19}(OCH_2(CH_3)CH_2)_{n5}OH$$

wherein $R^{19}$ is straight or branched, saturated or unsaturated fatty alcohol which may be synthetic or natural with a C chain length in the range of 8 to 40, preferably 9 to 30 and more preferably 9 to 24 and n5 is a number in the range of 1 to 40, preferably 3 to 30.

Suitable non-limiting examples are PPG-3 Caprylyl ether, PPG-5 Caprylyl ether, PPG-10 Caprylyl ether, PPG-10 Cetyl ether, PPG-20 Cetyl ether, PPG-28 Cetyl ether, PPG-30 Cetyl ether, PPG-7 Lauryl ether, PPG-10 Lauryl ether, PPG-10 Oleyl ether, PPG-20 Oleyl ether, PPG-23 Oleyl ether, PPG-30 Oleyl ether, PPG-11 Stearyl ether and PPG-15 Stearyl ether.

Further suitable nonionic surfactants are polyethylene glycol fatty acid esters of the following general structure

$$R^{20}C(O)(OCH_2CH_2)_{n6}OH$$

wherein $R^{20}$ is straight or branched, saturated or unsaturated alkyl group which may be synthetic or natural with a C chain length in the range of 7 to 39, preferably 9 to 29 and more preferably 9 to 23 and n6 is a number in the range of 5 to 40, preferably 9 to 30.

Suitable non-limiting examples are PEG-8 Behenate, PEG-8 Caprate, PEG-8 Caprylate, PEG-5 Cocoate, PEG-8 Cocoate, PEG-9 Cocoate, PEG-10 Cocoate, PEG-15 Cocoate, PEG-6 Isopalmitate, PEG-6 Isostearate, PEG-8 Isostearate,

PEG-9 Isostearate, PEG-10 Isostearate, PEG-12 Isostearate, PEG-20 Isostearate, PEG-30 Isostearate, PEG-40 Isostearate, PEG-6 Laurate, PEG-8 Laurate, PEG-9 Laurate, PEG-10 Laurate, PEG-12 Laurate, PEG-14 Laurate, PEG-20 Laurate, PEG-30 Laurate, PEG-8 Myristate, PEG-20 Myristate, PEG-5 Oleate, PEG-6 Oleate, PEG-7 Oleate,

PEG-8 Oleate, PEG-9 Oleate, PEG-10 Oleate, PEG-11 Oleate, PEG-12 Oleate, PEG-15 Oleate, PEG-20 Oleate, PEG-30 Oleate, PEG-32 Oleate, PEG-6 Palmitate, PEG-18 Palmitate, PEG-20 Palmitate, PEG-5 Stearate, PEG-6 Stearate, PEG-7 Stearate, PEG-8 Stearate, PEG-9 Stearate, PEG-10 Stearate, PEG-12 Stearate, PEG-14 Stearate, PEG-15 Stearate, PEG-20 Stearate, PEG-25 Stearate, PEG-30 Stearate, PEG-35 Stearate and PEG-40 Stearate.

Further suitable nonionic surfactants are polypropylene glycol fatty acid esters of the following general structure

$$R^{21}C(O)(OCH_2(CH_3)CH_2)_{n8}OH$$

wherein $R^{21}$ is straight or branched, saturated or unsaturated alkyl group which may be synthetic or natural with a C chain length in the range of 7 to 39, preferably 9 to 29 and more preferably 9 to 23 and n8 is a number in the range of 1 to 40, preferably 9 to 30.

Suitable non-limiting examples are PPG-15 Isostearate, PPG-9 Laurate, PPG-26 Oleate and PPG-36 Oleate.

Further suitable nonionic surfactants are polyethylene glycol and polypropylene glycol ether of fatty alcohols of the following general structure

$$R^{22}(OCH_2(CH_3)CH_2)_{n9}(OCH_2CH_2)_{n10}OH$$

wherein $R^{22}$ is straight or branched, saturated or unsaturated alkyl group which may be synthetic or natural with a C chain length in the range of 7 to 39, preferably 9 to 29 and more preferably 9 to 23 and n9 and n10 may be the same or different and are a number in the range of 1 to 40.

Further suitable nonionic surfactants are ethoxylated triglycerides. Well known and commonly used examples are ethoxylated castor oil such as PEG-40 hydrogenated castor oil or and PEG-60 hydrogenated castor oil.

Preferably the non-ionic surfactant(s) are selected from linear or branched, saturated or unsaturated fatty alcohols with C6 to C22 being modified with 3-30 repeating units of ethylene oxide and/or propylene oxide.

Suitable anionic surfactants are selected from compounds according to the structure

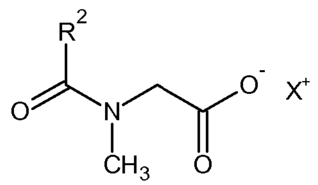

Formula II

wherein $R^2$ is a straight or branched, substituted or unsubstituted, saturated or unsaturated alkyl chain with a carbon number of $C_9$ to $C_{21}$, preferably $R^2$ is a straight alkyl chain with a carbon number of $C_9$ to $C_{17}$, and $X^+$ is a cation selected from sodium, potassium, magnesium and ammonium ions, which are compounds based on the amino acid sarcosin which are commonly known as sarcosinates.

Suitable compounds are, for example, cocoyl sarcosinate and its salts, lauroyl sarcosinate and its salts, myristoyl sarcosinate and its salts, stearoyl sarcosinate and its salts, oleoyl sarcosinate and its salts, palmitoyl sarcosinate and its salts. Salts are formed with cations selected from sodium, potassium, magnesium, and ammonium ions.

The preferred surfactant according to the structure of formula II is sodium lauroyl sarcosinate.

Suitable anionic surfactants are alkyl sulfate or preferably ethoxylated alkyl ether sulfate surfactant or mixtures thereof with an alkyl chain length of $C_{10}$ to $C_{22}$.

Suitable surfactants are laureth sulfates, coceth sulfate, pareth sulfate, capryleth sulphate, myreth sulfate, oleth sulfate, deceth sulfate, trideceth sulfate, coco sulphate, $C_{10}$-$C_{16}$ alkyl sulphate, $C_{11}$-$C_{15}$ alkyl sulphate, $C_{12}$-$C_{18}$ alkyl sulphate, $C_{12}$-$C_{15}$ alkyl sulphate, $C_{12}$-$C_{16}$ alkyl sulphate, $C_{12}$-$C_{13}$ alkyl sulfate, lauryl sulphate, myristyl sulphate, palm kernel sulphate, cetearyl sulfate, cetyl sulphate, decyl sulphate, oleyl sulphate, behenyl sulphate and/or their salts. All of the aforementioned anionic surfactants may or may not be ethoxylated at various degrees.

Cations for the surfactants may be selected from sodium, potassium, magnesium and/or ammonium.

Suitable anionic surfactant is sodium laureth sulfate with 1-5 ethylene oxide units.

The amphoteric surfactant may be selected from compounds according to the general structure(s) III and/or IV

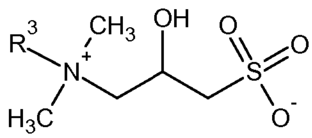

and/or

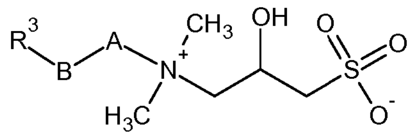

Formula III

Formula IV

wherein $R^3$ is a straight or branched, saturated or unsaturated, substituted or unsubstituted alkyl chain with a carbon number of $C_{10}$ to $C_{22}$, preferably $R^3$ is a straight alkyl chain with a carbon number of $C_{10}$ to $C_{16}$, A is a straight alkyl chain with a carbon number of $C_1$ to $C_6$ or a branched alkyl chain with a carbon number of $C_3$ to $C_6$, preferably A is a linear alkyl chain with a carbon number of $C_3$, and B is an amide or an ester group.

Suitable compounds are known as hydroxysultain surfactants, such as cocoamidopropyl hydroxysultaine, laurylamidopropyl hydroxysultaine, erucamidopropyl hydroxysultaine, lauryl hydroxysultaine, and cocoyl hydrodroxysultaine.

Other suitable zwitterionic surfactants are known as betaines. A suitable example is cocoamidopropylbetaine

The preferred amphoteric/zwitterionic surfactant is/are lauryl hydroxysultaine and cocoamidopropylbetaine.

The composition may comprise amphoteric and/or zwitterionic surfactants at a total concentration in the range of 0.1% to 2%, preferably 0.25% to 1.75%, more preferably 0.5% to 1.5% by weight, calculated to the total of the composition.

The composition may further comprise cationic surfactants of quaternary ammonium structure according to

$$R_5\ R_6\ R_7\ R_8\ N$$

wherein $R_5$ is an alky chain having C length of 8 to 30 which may be saturated or unsaturated, straight or branched, $R_6$ is an alkyl chain having C length of 1 to 30 which may be saturated or unsaturated, straight or branched, $R_5$ and $R_6$ additionally may take the structures of

$$R_9C(O)O(CH_2)_{n1} \text{ or } R_{10}C(O)NH(CH_2)_{n1}$$

wherein $R_9$ is an alkyl chain with a C length of 7 to 29 which may be saturated or unsaturated, straight or branched and n1 is a number between 1 and 4,

$R_7$ and $R_8$ are same or the different alkyl chain with a C length of 1 to 4 which may be straight or branched (only for $C_3$ and $C_4$), wherein all alkyl chains may comprise one or more substituents such as hydroxyl- or (poly)-ethoxy groups.

Anions for the cationic surfactants may be selected from chloride, sulfate, or nitrate.

The total concentration of surfactants may be in the range of 0.1% to 10% by weight, preferably 0.5% to 7.5% by weight, more preferably 1% to 5% by weight, calculated to the total of the composition.

The composition of the present invention further comprises one or more thickeners. In a preferred embodiment of the present invention, the composition comprises one or more thickening polymers selected from anionic, nonionic, cationic and amphoteric polymers, preferably selected from polymers resulting in a solution and/or dispersion with a viscosity of at least 500 mPa•s measured at a polymer concentration of 1% by weight in water and at 20°C with a Brookfield viscometer, such as at 10 rpm for 1 min, with an appropriate spindle.

Suitable polymers are cellulose polymers, alginates, polysaccharides and acrylic acid polymers, preferably methyl cellulose, ethyl cellulose, hydroxyethylcellulose, methylhydroxyethylcellulose, methylhydroxypropylcellulose, carboxymethyl cellulose, alginic acids, sodium alginates, ammonium alginates, calcium alginates, gum arabic, guar gum or xanthan gum, dehydroxanthan gum or acrylic acid polymers known with the CTFA adopted name Carbomer and its derivatives.

The preferred polymers are dehydroxanthan gum, xanthan gum, and polymeric anionic thickeners, namely Carbomer and its derivatives.

Of particular advantageous use are thickeners which are commonly known as associative thickeners. Preferred are copolymers and/or crosspolymers which comprise an acrylate and/or methacrylate monomer unit and at least one more hydrophobic unit such as alkyl chains. Examples are acrylates/c10-30 alkyl acrylate crosspolymer, acrylates/steareth-20 methacrylate copolymer, acrylates/stearyl acrylate/dimethicone methacrylate copolymer, acrylates/beheneth-25 methacrylate copolymer, acrylates/lauryl acrylate/stearyl acrylate/ ethylamine oxide methacrylate copolymer.

The composition preferably comprises thickening agents at a total concentration in the range of 0.1% to 5%,

preferably, 0.2% to 3%, more preferably 0.25% to 2.5% and most preferably 0.3% to 2% by weight calculated to the total of the composition.

The composition of the present invention further comprises hair direct dyes selected from non-ionic, cationic and/or anionic hair direct dyes.

Suitable anionic direct dyes are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium. Among those, the most preferred anionic dyestuffs are Acid Red 52, DC Violet 2, DC Red 33, DC Orange 4, DC Red 27, DC Yellow 10, HC Blue 18, HC Red 18, and HC Yellow 16.

Suitable cationic dyes are in principle those available on the market for cosmetic hair colouring applications. For this purpose, special reference is made to the PCT application WO 95/15144 of Ciba-Geigy AG. Some examples to those are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14, Basic Yellow 57, Basic red 51, Basic Yellow 87, HC Blue 17 and Basic Orange 31. The most preferred ones are Basic red 51, Basic Yellow 87 and Basic Orange 31 sold by BASF, HC Blue 17, Basic Blue 124.

Suitable neutral dyes including nitro dyes are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

The most preferred direct dyes are HC Red 18, HC Yellow 16, and HC Blue 17.

The composition may comprise one or more hair direct dye at a total concentration of 0.001% to 10% by weight, preferably 0.005% to 7.5% by weight, and more preferably 0.01% to 5% by weight, calculated to the total of the composition. The composition can also comprise a mixture of several direct dyes, i.e., an anionic, a cationic and/or non-ionic ones. In such a case the dyes may be mixed at any ratio with each other.

The composition further comprises stabilizing agents. Suitable stabilizing agents are 8-hydroxy-quinoline and/or its salts and/or its hydrates and/or salicylic acid and/or 4-hydroxyacetanilid at a concentration in the range of 0.001% to 0.25% by weight, preferably 0.01% to 0.20% by weight, more preferably 0.02% to 0.15% by weight, calculated to the total of the composition.

The composition of the present invention is mixed with a second composition comprising an alkalizing agent. The second composition is a powder composition or a liquid composition. Although being a powder composition, it may comprise liquid compounds at a low amount. For liquid compositions, the composition may be a solution or emulsion wherein emulsion form is preferred.

The second composition comprises non-ammonia alkalizing agents such as 2-amino-2-methylpropanol, sodium metasilicate, and organic alkyl and/or alkanol amine salt(s) according to the general structure

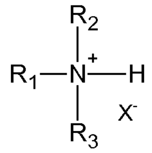

Formula I

wherein R1, R2, and R3 are independently selected from H, linear C1-C6 alkyl which may be substituted with one hydroxyl group, or branched C3-C12 alkyl or alkanol, wherein at least one of R1, R2, or R3 is different from H, and X- is an anion.

The organic amine according to the structure above may be selected from mono- and/or diethanolamine, butyl ethanolamine, butyl diethanolamine, dibutyl ethanolamine, methylethanolamine, triethanolamine, N-lauryl diethanolamine, diisopropanolamine, dimethyl isopropanolamine, isopropanolamine, triisopropanolamine, isobutanolamine.

The counterion X- is selected from chloride and/or hydrogen chloride, nitrate, sulphate, phosphate, hydrogenphosphate, dihydrogenphosphate, citrate, acetate, sulphite, benzoate, salicylate. The most preferred organic amine salt is mono- or diethanolamine hydrochloride.

The concentration of alkalizing agents in the second composition is preferably in the range of 1% to 20% by weight, more preferably 2% to 15% by weight, calculated to the total of the composition.

In one embodiment of the present invention, the second composition comprises non-polymeric salts of $NH_4^+$ at a total concentration below 3% by weight, calculated to the total of the second composition.

It is a preferred embodiment of the present invention that the second composition is free of non-polymeric salts of $NH_4^+$.

The second composition further comprises oxidative hair dyes.

Suitable non-limiting examples of oxidative dye precursor classes are p-phenylendiamines, p-aminophenols, and heterocyclic compounds such as diaminopyrazols and substituted pyrimidines, and suitable coupling substances are resorcinols, m-aminophenols, m-phenylendiamines, pyridines and its derivatives, and naphthols.

Non-limiting examples of the oxidative dye precursor compounds are p-phenylenediamine, p-aminophenol, 2,5-diamino-toluene, 2-n-propyl or 2-ethyl-p-phenylenediamine, 2,6-di-methyl-p-phenylene- diamine, 2-(2,5-diaminophenyl) ethanol, 1-amino-4-bis-(2'-hydroxy-ethyl)amino- benzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene, 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 3,5-diamino-1,2,4-triazole, 4-aminophenol and the derivatives thereof such as 4-amino-3-methylphenol, 2-chloro-4-aminophenol, 2,6-dichloro-4-aminophenol, 2,4-diamino-phenol, 2,6-dibromo-4-aminophenol, tetraamino pyrimidines, triaminohydroxy pyrimidines, diaminomono- and -dihydroxy pyrimidines, aminotriazines, 5-amino salicylic acid and/or 1,2,4-triamino benzene, 2,5-diaminopyridine, 2,3-diaminopyridine, 2,6-diaminopyridine, 3-amino-2-methyl amino-6-methoxypyridine, 2-dimethyl-5-aminopyridine, 2-dimethyl aminoethyl-3-hydroxypyridine, 2-amino-4,6-dimethyl pyridine, 2-amino-3-hydroxypyridine, 3-amino-2(β-hydroxyethyl amino)-6-methoxy pyridine, 2,6-dimethyl amino-5-aminopyridine, 2-di(hydroxyethyl) amino-5-aminopyridine, 2-hydroxyethyl amino-5-aminopyridine, 4-hydroxy-2,5,6-triaminopyrimidine and/or the water-soluble salts thereof, and mixture thereof.

The total concentration of the dye precursors (developing substances) customarily ranges between 0.001 to 5%, preferably 0.01 to 4% and more preferably 0.05 to 3%, and most preferably 0.1 to 2% by weight, calculated to the total of the second composition.

Suitable non-limiting examples of the coupling substance if present in the second composition are 5-amino-2-methylphenol, 2-methyl-5-hydroxyethylaminophenol, 2,4,-diaminophenoxyethanol, 2-amino-4-hydroxyethylaminoanisol, 2-methyl-5-amino-6-chlorphenol, 1,3-bis(2,4-diaminophenoxy)propane, 2-bis(2-hydroxyethyl)aminotoluene, 2-amino-5-methylphenol, resorcinol, 2-methyl-resorcinol, 4-chlororesorcinol, 2-amino-4-chlorophenol, 5-amino-4-methoxy-2-methylphenol, 2-aminophenol, 3-amino-phenol, 1-methyl-2-hydroxy-4-aminobenzene, 3-N,N-dimethyl aminophenol, 2,6-dihydroxy-3,5-dimethoxypyridine, 5-amino-3-methylphenol, 6-amino-3-methylphenol, 1,3-diamino- benzene, 1-amino-3-(2'-hydroxyethylamino)benzene, 1-amino-3-[bis(2'-hydroxy-ethyl) amino]benzene, α-naphthol, 4,6-dichlororesorcinol, 1,3-diamino-toluene,

4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1,2-methyldioxy benzene, 2,4-diamino-3-chlorophenol, 5-amino-2-methoxyphenol and/or 1-methoxy-2-amino-4-(2'-hydroxyethyl amino)benzene or the water-soluble salts thereof and mixture thereof.

Coupling substance(s) in the second composition as reaction partners for the developing substance(s) are present in approximately the same molecular proportions as the developing substances, i.e. at a total concentration in the range of 0.001 to 5%, preferably 0.01 to 4% and more preferably 0.05 to 3%, and most preferably 0.1 to 2% by weight, calculated to the total of the second composition.

The second composition comprises hair direct dyes. Suitable hair direct dyes are identical to the ones disclosed for the composition according to the present invention.

Prior to application onto hair, the composition of the present invention must be mixed with a second composition to yield a ready-to-use composition with a pH in the range of 7 to 12. Preferred mixing ratios by weight of the composition according to the present invention to second composition are 1:1 to 2:1, but other mixing ratios are possible as well.

The following examples are to illustrate the invention, but not to limit it.

## EXAMPLES

### EXAMPLE 1

The following powder compositions were prepared:

| Ingredient | Powder composition A |
|---|---|
| Paraffin oil | 10.0 |
| Corn starch | 7.5 |
| Sodium bicarbonate | 2.0 |
| Sodium metasilicate | 2.0 |
| 2-amino-2-methylpropanol | 0.5 |
| HC Yellow 16 | 0.5 |
| HC Blue 18 | 0.33 |
| HC Red 18 | 0.15 |
| Diatomaceous earth | Ad 100.0 |

The powder composition from above was mixed with 1) water, or 2) a solution of monoethanolamine hydrochloride at 1% by weight in water. The mixing ratio by weight of the powder composition to the aqueous composition was 1:2. The resulting pH of the mixture with water was 10, whereas the mixture with the monoethanolamine salt composition resulted in a pH of 9.71.

2 g of the resulting ready-to-use mixtures were applied onto goat hair streaks purchased from Fischbach + Miller, Laupheim, Germany. The compositions were left onto hair for 30 min at room temperature. Then the compositions were rinsed-off with water and the hair streaks were shampooed. Hair streaks were then blow-dried.

Color parameters were measured prior to coloring and after bleaching by spectrophotometrical analysis with a Datacolor 45G CT instrument delivered from Datacolor Inc., Lawrenceville, NJ, USA. Based on the CIE*Lab color space results obtained by the measurements, $\Delta E_{ab}$ values for color difference were calculated according to equation (1):

$$\Delta E_{ab} = \sqrt{(L_2 - L_1) + (a_2 - a_1) + (b_2 - b_1)} \qquad \text{Equation 1}$$

The resulting $\Delta E_{ab}$ values for the water mixture was 52.27, whereas the monoethanolamine salt composition yielded 53.94. As the $\Delta E_{ab}$ value is higher for the latter composition, the monoethanolamine salt composition delivered a more intensive color.

### EXAMPLE 2

The following oxidizing compositions were prepared by conventional formulation methods:

| Ingredient | Inventive composition A | Inventive composition B | Comparative composition |
|---|---|---|---|
| Cetearyl alcohol | 5.0 | 5.0 | 5.0 |
| Ceteareth-30 | 2.5 | 2.5 | 2.5 |
| Monoethanolamine HCl | 5.0 | - | - |
| Diethanolamine HCl | - | 5.0 | - |
| Hydrogen peroxide | 12.0 | | |
| Phosphoric acid | q.s. pH 2.0 | | |
| Water | Ad 100.0 | | |

The following powder compositions were prepared:

| Ingredient | Powder composition A | Powder composition B |
|---|---|---|
| Paraffin oil | 10.0 | 10.0 |
| Corn starch | 7.5 | 7.5 |
| Sodium bicarbonate | 2.0 | 2.0 |
| Sodium metasilicate | 2.0 | 2.0 |
| 2-amino-2-methylpropanol | 0.5 | 0.5 |
| Monoethanolamine HCl | - | 5.0 |
| HC Yellow 16 | 0.5 | 0.5 |
| HC Blue 18 | 0.33 | 0.33 |
| HC Red 18 | 0.15 | 0.15 |
| Diatomaceous earth | Ad 100.0 | |

The following mixtures were prepared:

| Ready-to-use compositions | Aqueous oxidizing composition | Powder composition | Mixture pH |
|---|---|---|---|
| 1 | 2 parts inventive composition A | 1 part powder composition A | 8.75 |
| 2 | 2 parts inventive composition B | 1 part powder composition A | 8.24 |
| 3 | 2 part comparative composition | 1 part powder composition B | 8.36 |

2 g of the resulting ready-to-use mixtures were applied onto bleached human hair streaks (Caucasian, 21 cm long) purchased from Fischbach + Miller, Laupheim, Germany. The compositions were left onto hair for 30 min at room temperature. Then the compositions were rinsed-off with water and the hair streaks were shampooed.

Hair streaks were then blow-dried.

Color parameters were measured as described for example 1.

The following results were obtained:

| Ready-to-use compositions | L | a | b | $\triangle E_{ab}$ |
|---|---|---|---|---|
| 1 prior to coloring | 81.93 | 0.09 | 13.85 | 59.28 |
| 1 upon coloring | 28.17 | 16.21 | -5.23 | |
| 2 prior to coloring | 81.93 | 0.09 | 13.85 | 58.69 |
| 2 upon coloring | 29.32 | 17.32 | -5.64 | |
| 3 prior to coloring | 81.93 | 0.09 | 13.85 | 55.97 |
| 3 upon coloring | 28.52 | 16.24 | 9.36 | |

As a result, the inventive compositions delivered more intensive coloring in comparison to the comparative compositions.

The following examples are within the scope of the invention.

**EXAMPLE** 3

| | % by weight |
|---|---|
| Stearyl alcohol | 7.5 |
| Isopropyl palmitate | 5.0 |

(continued)

| | % by weight |
|---|---|
| PEG-40 hydrogenated castor oil | 3.0 |
| 8-hydroxy-quinoline sulfate monohydrate | 0.03 |
| Monoethanolamine hydrochloride | 3.0 |
| Diethanolamine hydrochloride | 1.0 |
| Ammonium sulfate | 0.5 |
| Ammonium chloride | 0.5 |
| Hydrogen peroxide | 9.0 |
| Phosphoric acid | q.s. ad pH 4.0 |
| Water | ad 100.0 |

The composition is employed upon mixture with either powder composition from example 1 in the same ratio.

**EXAMPLE** 4

| | % by weight |
|---|---|
| Behenyl alcohol | 5.0 |
| Sunflower oil | 10.0 |
| Steareth-20 | 2.0 |
| Sodium lauryl sulfate | 1.0 |
| Salicylic acid | 0.02 |
| 4-hydroxyacetanilid | 0.02 |
| Monoethanolamine hydrochloride | 1.5 |
| HC Blue 18 | 0.2 |
| Dehydroxanthan gum | 0.5 |
| Hydrogen peroxide | 9.0 |
| Phosphoric acid | q.s. ad pH 2.0 |
| Water | ad 100.0 |

The composition is employed upon mixture with either powder composition from example 1 in the same ratio.

**EXAMPLE 5**

| | % by weight |
|---|---|
| Stearyl alcohol | 8.0 |
| Pareth-20 | 2.0 |
| Salicylic acid | 0.02 |
| 4-hydroxyacetanilid | 0.02 |
| Triethanolamine hydrochloride | 5.0 |
| Monoethanolamine hydrochloride | 1.0 |
| HC Red 18 | 0.2 |
| Acrylates copolymer (acrylates/steareth-20 methacrylate copolymer) | 0.5 |
| Hydrogen peroxide | 6.0 |
| Phosphoric acid | q.s. ad pH 4.5 |
| Water | ad 100.0 |

The composition is employed upon mixture with either powder composition from example 1 in the same ratio.

**EXAMPLE 6**

**Oxidative composition**

| | % by weight |
|---|---|
| Stearyl alcohol | 8.0 |
| Pareth-20 | 2.0 |
| Salicylic acid | 0.02 |
| 4-hydroxyacetanilid | 0.02 |
| Monoethanolamine hydrochloride | 5.0 |
| Ammonium chloride | 1.0 |
| HC Red 18 | 0.2 |
| Acrylates copolymer (acrylates/steareth-20 methacrylate copolymer) | 0.5 |
| Hydrogen peroxide | 12.0 |
| Phosphoric acid | q.s. ad pH 4.5 |
| Water | ad 100.0 |

**Dyeing composition**

| | % by weight |
|---|---|
| Cetearyl alcohol | 10.0 |
| Cocamide MEA | 4.0 |
| Sodium lauryl sulphate | 1.5 |
| Propylene glycol | 2.0 |
| Cetyltrimonium chloride | 0.5 |
| 2,5,6-Triamino-4-hydroxypyrimidine sulfate | 0.01 |
| 2,5-Diaminotoluene sulfate | 0.55 |
| 4-Chlororesorcinol | 0.17 |
| Resorcinol | 0.05 |
| 3-Aminophenol | 0.03 |
| Sodium sulfite | 1.0 |
| Aminomethyl propanol | 2.0 |
| Ammonium hydroxide | q.s. to pH 10.0 |
| Fragrance, preservative | q.s. |
| Water | to 100 |

The oxidative composition and dyeing composition were mixed a ratio 1:1.

## Claims

1. An aqueous cosmetic composition for bleaching and/or dyeing of keratin fibers, preferably human keratin fibers, more preferably human hair **characterized in that** it comprises an organic alkyl and/or alkanol amine salt according to the general structure

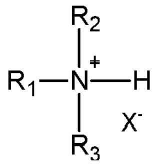

wherein R1, R2, and R3 are independently selected from H, linear C1-C6 alkyl which may be substituted with one hydroxyl group, or branched C3-C12 alkyl or alkanol,
wherein at least one of R1, R2, or R3 is different from H, and X- is an anion at a total concentration in the range of 0.5 to 15% by weight, calculated to the total weight of the composition, wherein the composition has a pH in the range of 1 to 5,
wherein the composition is an emulsion,
wherein the composition comprises lipophilic compounds selected from natural and/or vegetable oils, petrolatum-based compounds, linear or branched, saturated or unsaturated fatty alcohols with C12 to C22, and fatty acid esters consisting of linear or branched, saturated or unsaturated fatty acids with C12 to C22 being esterified with linear or branched primary alcohols with C3 to C12,
and wherein the composition is different from the one comprising 2.5% by weight hydrogen peroxide, 0.2% by weight Polyquaternium 87, 0.2% by weight monoethanolamine, citric acid q.s. to pH 3.0, 0.3% by weight PEG-40-Hydrogenated castor oil, 0.3% by weight fragrance, and water q.s. to 100.

2. The composition according to claim 1 **characterized in that** the organic amine is selected from mono- and/or diethanolamine, butyl ethanolamine, butyl diethanolamine, dibutyl ethanolamine, methylethanolamine, triethanolamine, N-lauryl diethanolamine, diisopropanolamine, dimethyl isopropanolamine, isopropanolamine, triisopropanolamine, isobutanolamine.

3. The composition according to any of the preceding claims **characterized in that** the salt of organic amine is mono- or diethanolamine hydrochloride.

4. The composition according to any of the preceding claims **characterized in that** the composition comprises the salt of organic amine at a total concentration of 0.5% to 12% by weight, more preferably 1% to 10% by weight, calculated to the total of the composition.

5. The composition according to any of the preceding claims **characterized in that** it comprises one or more non-polymeric salts of $NH_4^+$ at a concentration of 0.01% to 3% by weight, calculated to the total of the composition.

6. The composition according to any of the claims 1 to 4 **characterized in that** it is free of non-polymeric salts of $NH_4^+$.

7. The composition according to any of the preceding claims **characterized in that** it comprises hydrogen peroxide at a total concentration in the range of 1% to 20% by weight, preferably in the range of 1% to 12% by weight, more preferably in the range of 3% to 9% by weight, calculated to the total of the composition.

8. The composition according to any of the preceding claims **characterized in that** it comprises 8-hydroxy-quinoline and/or its salts and/or its hydrates and/or salicylic acid and/or 4-hydroxyacetanilid at a concentration in the range of 0.001% to 0.25% by weight, preferably 0.01% to 0.20% by weight, more preferably 0.02% to 0.15% by weight, calculated to the total of the composition.

9. The composition according to any of the preceding claims **characterized in that** the composition comprises direct dyes selected from cationic, anionic, and non-ionic direct dyes.

10. The composition according to any of the preceding claims **characterized in that** composition comprises direct dyes selected from HC Red 18, HC Yellow 16, and HC Blue 17.

11. A process for bleaching and/or dyeing of keratin fibers, preferably human keratin fibers, more preferably human hair, **characterized in that** it comprises the following steps:

a) mixing the composition according to any of the claims 1 to 10 with a composition comprising an alkalizing agent to form a ready-to-use mixture having a pH in the range of 7 to 12,

b) applying the mixture onto keratin fibers and leaving it for 1 min to 45 min,
c) rinsing-off the mixture,
d) optionally shampooing the keratin fibers,
e) optionally drying the keratin fibers.

**12.** Use of the composition as defined in the claims 1 to 10 for dyeing and/or bleaching keratin fibers, preferably human keratin fibers, more preferably human hair.

**13.** A kit-of-parts comprising in a separately packed container the composition as defined in the claims 1 to 10 and in at least one other separately packed container a composition comprising an alkalizing agent.

**Patentansprüche**

**1.** Wässrige kosmetische Zusammensetzung zum Bleichen und/oder Färben von Keratinfasern, vorzugsweise menschlichen Keratinfasern, besonders bevorzugt menschlichem Haar, **dadurch gekennzeichnet, dass** sie ein organisches Alkyl- und/oder Alkanolamin-Salz der folgenden allgemeinen Struktur umfasst

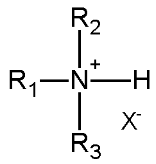

worin R1, R2 und R3 unabhängig voneinander ausgewählt sind aus H, linearem C1-C6-Alkyl, das mit einer Hydroxylgruppe substituiert sein kann, oder verzweigtem C3-C12-Alkyl oder Alkanol, wobei mindestens eines von R1, R2 oder R3 von H verschieden ist, und X- ein Anion in einer Gesamtkonzentration im Bereich von 0.5 bis 15 Gew.-%, berechnet auf das Gesamtgewicht der Zusammensetzung, ist, wobei die Zusammensetzung einen pH-Wert im Bereich von 1 bis 5 aufweist,
wobei die Zusammensetzung eine Emulsion ist,
wobei die Zusammensetzung lipophile Verbindungen, ausgewählt aus natürlichen und/oder pflanzlichen Ölen, Verbindungen auf Petrolatumbasis, linearen oder verzweigten, gesättigten oder ungesättigten Fettalkoholen mit C12 bis C22 und Fettsäureestern, bestehend aus linearen oder verzweigten, gesättigten oder ungesättigten Fettsäuren mit C12 bis C22, die mit linearen oder verzweigten primären Alkoholen mit C3 bis C12 verestert sind, umfasst,
und wobei sich die Zusammensetzung von derjenigen unterscheidet, die 2.5 Gew.-% Wasserstoffperoxid, 0.2 Gew.-% Polyquaternium 87, 0.2 Gew.-% Monoethanolamin, Zitronensäure q.s. bis pH 3.0, 0.3 Gew.-% PEG-40-hydriertes Rizinusöl, 0.3 Gew.-% Duftstoff und Wasser q.s. bis 100 enthält.

**2.** Zusammensetzung nach Anspruch 1, **dadurch gekennzeichnet, dass** das organische Amin ausgewählt ist aus Mono- und/oder Diethanolamin, Butylethanolamin, Butyldiethanolamin, Dibutylethanolamin, Methylethanolamin, Triethanolamin, N-Lauryldiethanolamin, Diisopropanolamin, Dimethylisopropanolamin, Isopropanolamin, Triisopropanolamin, Isobutanolamin.

**3.** Zusammensetzung nach einem der vorhergehenden Ansprüche, **dadurch gekennzeichnet, dass** das Salz des organischen Amins Mono- oder Diethanolaminhydrochlorid ist.

**4.** Zusammensetzung nach einem der vorhergehenden Ansprüche, **dadurch gekennzeichnet, dass** die Zusammensetzung das Salz des organischen Amins in einer Gesamtkonzentration von 0.5 bis 12 Gew.-%, vorzugsweise von 1 bis 10 Gew.-%, bezogen auf die Gesamtmenge der Zusammensetzung, enthält.

**5.** Zusammensetzung nach einem der vorangehenden Ansprüche, **dadurch gekennzeichnet, dass** sie ein oder mehrere nichtpolymere Salze von NH4+ in einer Konzentration von 0.01 bis 3 Gew.-%, berechnet auf die Gesamtmenge der Zusammensetzung, enthält.

**6.** Zusammensetzung nach einem der Ansprüche 1 bis 4, **dadurch gekennzeichnet, dass** sie frei von nichtpolymeren Salzen von NH4+ ist.

7. Zusammensetzung nach einem der vorhergehenden Ansprüche, **dadurch gekennzeichnet, dass** sie Wasserstoffperoxid in einer Gesamtkonzentration im Bereich von 1 bis 20 Gew.-%, vorzugsweise im Bereich von 1 bis 12 Gew.-%, besonders bevorzugt im Bereich von 3 bis 9 Gew.-%, bezogen auf die Gesamtzusammensetzung, enthält.

8. Zusammensetzung nach einem der vorhergehenden Ansprüche, **dadurch gekennzeichnet, dass** sie 8-Hydroxychinolin und/oder dessen Salze und/oder dessen Hydrate und/oder Salicylsäure und/oder 4-Hydroxyacetanilid in einer Konzentration im Bereich von 0.001 bis 0.25 Gew.-%, vorzugsweise 0.01 bis 0.20 Gew.-%, besonders bevorzugt 0.02 bis 0.15 Gew.-%, bezogen auf die Gesamtzusammensetzung, enthält.

9. Zusammensetzung nach einem der vorhergehenden Ansprüche, **dadurch gekennzeichnet, dass** die Zusammensetzung Direktfarbstoffe, ausgewählt aus kationischen, anionischen und nichtionischen Direktfarbstoffen, enthält.

10. Die Zusammensetzung nach einem der vorhergehenden Ansprüche, **dadurch gekennzeichnet, dass** die Zusammensetzung Direktfarbstoffe enthält, die aus HC Red 18, HC Yellow 16 und HC Blue 17 ausgewählt sind.

11. Verfahren zum Bleichen und/oder Färben von Keratinfasern, vorzugsweise menschlichen Keratinfasern, besonders bevorzugt menschlichen Haaren, **dadurch gekennzeichnet, dass** es die folgenden Schritte umfasst:

   a) Mischen der Zusammensetzung nach einem der Ansprüche 1 bis 10 mit einer Zusammensetzung, die ein Alkalisierungsmittel enthält, um eine gebrauchsfertige Mischung mit einem pH-Wert im Bereich von 7 bis 12 zu bilden,
   b) Auftragen der Mischung auf die Keratinfasern und Einwirkenlassen für 1 min bis 45 min,
   c) Abspülen der Mischung,
   d) gegebenenfalls Shampoonieren der Keratinfasern,
   e) gegebenenfalls Trocknen der Keratinfasern.

12. Verwendung der Zusammensetzung gemäß den Ansprüchen 1 bis 10 zum Färben und/oder Bleichen von Keratinfasern, vorzugsweise menschlichen Keratinfasern, insbesondere menschlichen Haaren.

13. Kit, enthaltend in einem getrennt verpackten Behälter die Zusammensetzung nach einem der Ansprüche 1 bis 10 und in mindestens einem weiteren getrennt verpackten Behälter eine Zusammensetzung, die ein Alkalisierungsmittel enthält.

## Revendications

1. Composition cosmétique aqueuse pour la décoloration et/ou la teinture des fibres kératiniques, de préférence des fibres kératiniques humaines, plus préférentiellement des cheveux humains, **caractérisée par le fait qu'**elle comprend un sel organique d'alkyl et/ou d'alkanol amine selon la structure générale suivante

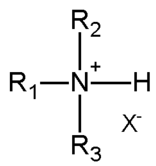

dans laquelle R1, R2 et R3 sont indépendamment choisis parmi H, alkyle linéaire en C1-C6 pouvant être substitué par un groupe hydroxyle, ou alkyle ramifié en C3-C12 ou alcanol, dans laquelle au moins un des R1, R2 ou R3 est différent de H, et X- est un anion à une concentration totale comprise entre 0.5 et 15% en poids, calculée par rapport au poids total de la composition, dans laquelle la composition a un pH compris entre 1 et 5, dans laquelle la composition est une émulsion,
dans laquelle la composition comprend des composés lipophiles choisis parmi les huiles naturelles et/ou végétales, les composés à base de pétrolatum, les alcools gras linéaires ou ramifiés, saturés ou insaturés en C12 à C22, et les esters d'acides gras constitués d'acides gras linéaires ou ramifiés, saturés ou insaturés en C12 à C22, estérifiés avec des alcools primaires linéaires ou ramifiés en C3 à C12,
et dans laquelle la composition est différente de celle comprenant 2.5% en poids de peroxyde d'hydrogène, 0.2% en poids de Polyquaternium 87, 0.2% en poids de monoéthanolamine, de l'acide citrique q.s. à pH 3.0.

0.3% en poids d'huile de ricin PEG-40-hydrogénée, 0.3% en poids de parfum et de l'eau q.s. à 100.

2. Composition selon la revendication 1, **caractérisée par le fait que** l'amine organique est choisie parmi la mono-et/ou la diéthanolamine, la butyléthanolamine, la butyldiéthanolamine, la dibutyléthanolamine, la méthyléthanolamine, la triéthanolamine, la N-lauryldiéthanolamine, la diisopropanolamine, la diméthylisopropanolamine, l'isopropanolamine, la triisopropanolamine, l'isobutanolamine.

3. Composition selon l'une quelconque des revendications précédentes, **caractérisée par le fait que** le sel d'amine organique est le chlorhydrate de mono ou de diéthanolamine.

4. La composition selon l'une des revendications précédentes est **caractérisée par le fait que** la composition comprend le sel d'amine organique à une concentration totale de 0.5% à 12% en poids, plus préférentiellement de 1% à 10% en poids, calculée par rapport au total de la composition.

5. La composition selon l'une des revendications précédentes est **caractérisée par le fait qu'**elle comprend un ou plusieurs sels non polymériques de $NH_4^+$ à une concentration de 0.01% à 3% en poids, calculée par rapport au total de la composition.

6. Composition selon l'une des revendications 1 à 4, **caractérisée par le fait qu'**elle est exempte de sels non polymériques de $NH_4^+$.

7. La composition selon l'une des revendications précédentes est **caractérisée par le fait qu'**elle comprend du peroxyde d'hydrogène à une concentration totale comprise entre 1% et 20% en poids, de préférence entre 1% et 12% en poids, plus préférentiellement entre 3% et 9% en poids, calculée par rapport au total de la composition.

8. La composition selon l'une des revendications précédentes est **caractérisée par le fait qu'**elle comprend la 8-hydroxy-quinoléine et/ou ses sels et/ou ses hydrates et/ou l'acide salicylique et/ou le 4-hydroxyacétanilide à une concentration comprise entre 0.001% et 0.25% en poids, de préférence entre 0.01% et 0.20% en poids, plus préférentiellement entre 0.02% et 0.15% en poids, calculée par rapport à la composition totale.

9. Composition selon l'une des revendications précédentes **caractérisée par le fait que** la composition comprend des colorants directs choisis parmi les colorants directs cationiques, anioniques et non ioniques.

10. La composition selon l'une des revendications précédentes est **caractérisée par le fait qu'**elle comprend des colorants directs choisis parmi le HC Red 18, le HC Yellow 16 et le HC Blue 17.

11. Procédé de décoloration et/ou de teinture de fibres kératiniques, de préférence de fibres kératiniques humaines, plus particulièrement de cheveux humains, **caractérisé en ce qu'**il comprend les étapes suivantes :

    a) mélanger la composition selon l'une des revendications 1 à 10 avec une composition comprenant un agent alcalinisant pour former un mélange prêt à l'emploi ayant un pH compris entre 7 et 12,
    b) appliquer le mélange sur les fibres kératiniques et le laisser agir pendant 1 à 45 minutes,
    c) rincer le mélange,
    d) éventuellement, shampouiner les fibres kératiniques,
    e) séchage éventuel des fibres kératiniques.

12. Utilisation de la composition telle que définie dans les revendications 1 à 10 pour la teinture et/ou le blanchiment des fibres kératiniques, de préférence des fibres kératiniques humaines, plus préférentiellement des cheveux humains.

13. Kit de pièces comprenant dans un récipient emballé séparément la composition définie dans les revendications 1 à 10 et dans au moins un autre récipient emballé séparément une composition comprenant un agent alcalinisant.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2476405 A
- EP 2931227 A
- EP 2177203 A
- US 20080060142 A
- US 20050125913 A
- US 5180399 A
- EP 70074 A
- JP 2015123019 A
- WO 9515144 A